Europäisches Patentamt

European Patent Office

Office européen des brevets

(19)

(11) **EP 0 570 336 B1**

(12) **EUROPÄISCHE PATENTSCHRIFT**

(45) Veröffentlichungstag und Bekanntmachung des Hinweises auf die Patenterteilung:
**24.01.2001   Patentblatt 2001/04**

(51) Int Cl.[7]: **C07D 223/22**

(21) Anmeldenummer: **93810326.4**

(22) Anmeldetag: **04.05.1993**

(54) **Hochtemperatur-Dehydrierungsverfahren zur Herstellung von 5H-Dibene b,f Azepin**

High temperature dehydration process for the production of 5H-dibenz(b,f)azepine

Procédé de déshydrogénation a haute température pour la fabrication de 5H-dibenz(b,f)azépine

(84) Benannte Vertragsstaaten:
**AT BE CH DE DK ES FR GB GR IE IT LI LU NL PT SE**

(30) Priorität: **13.05.1992  CH 153592**

(43) Veröffentlichungstag der Anmeldung:
**18.11.1993   Patentblatt 1993/46**

(73) Patentinhaber:
• **Novartis AG**
**4058 Basel (CH)**
Benannte Vertragsstaaten:
**BE CH DE DK ES FR GB GR IE IT LI LU NL PT SE**
• **Novartis-Erfindungen Verwaltungsgesellschaft m.b.H.**
**1235 Wien (AT)**
Benannte Vertragsstaaten:
**AT**

(72) Erfinder:
• **Aebli, Beat M.**
**CH-4004 Basel (CH)**
• **Monti, Daniel, Dr.**
**CH-4107 Ettingen (CH)**
• **Rusek, Milos**
**CH-4102 Binningen (CH)**

(56) Entgegenhaltungen:
**EP-A- 0 237 952          EP-A- 0 396 134**
**CH-A- 442 319           GB-A- 1 077 648**

• **PATENT ABSTRACTS OF JAPAN vol. 4, no. 42 (C-5)(524) 3. April 1980 & JP-A-55 017 330 (FUJI YAKUHIN KOGYO K.K.) 6. Februar 1980**
• **CHEMICAL AND PHARMACEUTICAL BULLETIN. Bd. 30 , 1982 , TOKYO JP Seiten 24 - 27 M. INOUE ET. AL. 'Catalytic Dehydrogenation of Iminodibenzyl to Iminostilbene on Mn2O3-SnO2'**

## Beschreibung

Die Erfindung betrifft ein neuartiges Verfahren zur Herstellung von Iminostilben durch Hochtemperatur-Dehydrierung von Iminodibenzyl an einem Eisenoxid/Kaliumsalz-Kontaktkatalysator in der Dampfphase.

Iminostilben, 5H-Dibenz[b,f]azepin ist ein wichtiges Zwischenprodukt für die Herstellung von 5-Carbamoyldibenz[b,f]azepin, welches unter der Freibezeichnung Carbamazepin grosse Bedeutung als antikonvulsiver Arzneimittelwirkstoff erlangt hat. Für die Überführung von Iminostilben in Carbamazepin sind mehrere Verfahren bekannt, die auf der direkten oder schrittweisen Einführung der Carbamoylgruppe in 5-Stellung des Azepinringes beruhen.

Für die Herstellung von Iminostilben steht eine Vielzahl von Verfahren zur Verfügung, von denen die Hochtemperatur-Dehydrierung von Iminodibenzyl in der Dampfphase an Eisenoxid-Kontakten besondere Bedeutung erlangt hat. Der derzeit technisch wichtigsten Variante dieses Verfahren gemäss CH-442,319 zufolge verwendet man einen Kontaktkatalysator, enthaltend "ausser Eisen-III-oxid, das am besten in Mengen von 30 -70 % verwendet wird, vorzugsweise noch Beimengungen von z.B. 1.5 - 3 Gew.-% $Cr_2O_3$, 10 - 15 Gew.-% CaO und im übrigen $K_2CO_3$" und arbeitet "bei Temperaturen von 300° bis 500° C, vorzugsweise von 400° bis 450° C". Höhere Temperaturen als 500° C führen, wie in CH-442,319 ausdrücklich hervorgehoben und für Reaktionstemperaturen von 550° und 600° C zahlenmässig belegt, "in untragbarem Ausmass oder sogar überwiegend" zu unerwünschten Acridinnebenprodukten.

Darüberhinaus hat das bekannte Verfahren den in CH-442,319 ausdrücklich erwähnten empfindlichen Nachteil, dass die Aktivität des Katalysators allmählich abnimmt, sodass dieser gemäss CH-442,319, Beispiel 2 bereits nach etwa 30-minütigem Einsatz regeneriert werden musste. Dies ist darauf zurückzuführen, dass ein nicht unerheblicher Anteil des eingesetzten Iminodibenzyls verharzt und in Form von teer- und kohleartigen Ablagerungen niedergeschlagen wird, die durch Ausbrennen des Katalysators periodisch entfernt werden müssen. Die in CH-442,319 angegebenen Ausbeuten können, wie insbesondere Beispiel 21 im Vergleich mit den Vergleichsbeispielen 1 und 2 zeigt, deshalb in der Praxis nicht erreicht werden. Die Verharzung von erheblichen Anteilen des eingesetzten Iminodibenzyls verringert aber nicht nur die Ausbeute an Iminostilben, sondern birgt auch ökologische und sicherheitstechnische Risiken infolge schwer kontrollierbarer oxidativer Nebenreaktionen. Insbesondere ist man zur periodischen Unterbrechung des Prozesses gezwungen, was eine kontinuierliche Betriebsweise verunmöglicht.

Der Erfindung lag deshalb die Aufgabe zugrunde, ein verbessertes Verfahren zur Herstellung von Iminostilben durch katalytische Hochtemperatur-Dehydrierung von Iminodibenzyl in der Dampfphase bereitzustellen, welches die oben beschriebenen und weitere Nachteile des bekannten Verfahrens vermeidet. Der erfindungsgemässe Vorschlag zur Lösung dieser Aufgabe beruht auf der überraschenden Feststellung, dass die periodische Regenerierung des Katalysators entbehrlich ist, wenn man einen Eisenoxid/Kaliumsalz-Kontaktkatalysator, enthaltend 35 bis 90 Gew.-% einer Eisenverbindung, berechnet als $Fe_2O_3$, und 7 bis 35 Gew.-% einer Kaliumverbindung, berechnet als $K_2O$, neben 0.0 bis 3.5 Gew.-% einer Chromverbindung, berechnet als $Cr_2O_3$, und gegebenenfalls üblichen Promotoren, verwendet.

Überraschend ist weiterhin, dass der erfindungsgemäss verwendete Katalysator bei der Hochtemperatur-Dehydrierung einer so hochmolekularen heterocyclischen Verbindung wie Iminodibenzyl autoregenerativ arbeitet und ein vorzügliches Aktivitäts- und Selektivitätsverhalten zeigt.

Überraschend ist insbesondere, dass, wie aus den Beispielen 12 bis 15 hervorgeht, entgegen dem durch CH-442,319 geschaffenen Vorurteil erfindungsgemäss gerade bei einer Reaktionstemperatur von 550° C und darüber der beste Umsatz und die höchste Ausbeute an Iminostilben erzielt wird.

Wie aus Beispiel 2 hervorgeht, wird erfindungsgemäss der Vorteil einer Lebensdauer des erfindungsgemäss verwendeten Kontaktkatalysators von über 1100 Stunden erzielt, ohne dass nennenswerte Mengen Iminodibenzyl durch unerwünschte oxidative Nebenreaktionen verlorengehen. Dadurch wird erfindungsgemäss die Möglichkeit einer kontinuierlichen Verfahrensweise eröffnet.

Das erfindungsgemässe kontinuierliche Verfahren zur Herstellung von Iminostilben durch Hochtemperatur-Dehydrierung von Iminodibenzyl an einem Eisenoxid/Kaliumsalz-Kontaktkatalysator in der Dampfphase ist dementsprechend dadurch gekennzeichnet, dass man einen Eisenoxid/Kaliumsalz-Kontaktkatalysator, enthaltend 35 bis 90, beispielsweise 44 bis 85, Gew.-% einer Eisenverbindung, berechnet als $Fe_2O_3$, und 7 bis 35, beispielsweise 9 bis 31, Gew.-% einer Kaliumverbindung, berechnet als $K_2O$, neben 0.0 bis 3.5 Gew.-% einer Chromverbindung, berechnet als $Cr_2O_3$, und gegebenenfalls üblichen Promotoren, verwendet, wobei der Promotorenanteil, bezogen auf den Kontaktkatalysator, höchstens 3,0 Gew % einer Calciumverbindung, berechnet als CaO, enthält.

Als weitere Promotoren kommen beispielsweise Magnesium-, Cer-, Molybdän-, Kobalt-, Vanadium- und Wolframverbindungen, beispielsweise in Form von Oxiden der genannten Metalle, insbesondere Wolfram-, Cer-, Vanadium- und Kobaltoxide, in Betracht.

Promotoren der genannten und ähnlicher Art können die Leistung des Katalysators zwar noch steigern, sind jedoch, wie die Beispiele 17 bis 20 zeigen nicht unbedingt erforderlich. Überhaupt ist sind die Mengenverhältnisse der Komponenten nicht kritisch, sofern nur die Eisen- und Kaliumanteile im Rahmen der oben gegebenen Definition bleiben.

Der erfindungsgemäss verwendete Kontaktkatalysator wird vorzugsweise, jedoch nicht zwingend, in einem adiabatischen Festbettreaktor in Kontakt mit Iminodibenzyldämpfen gebracht. Es können jedoch auch Rohrbündel-, Fliessbett- und Wirbelschichtreaktoren sowie andere dem Stand der Technik entsprechende Reaktortypen eingesetzt werden. Die Morphologie des erfindungsgemäss verwendeten Katalysators sollte den durch das Katalysatorbett strömenden Dämpfen möglichst wenig Widerstand bieten. Vorzugsweise verwendet man geometrische, beispielsweise zylindrische, Formkörper von etwa 1 bis 6 mm, insbesondere 1 bis 4 mm Länge und etwa 0.5 bis 5 mm, insbesondere 1 bis 3 mm Durchmesser, ferner Granulate mit einer durchschnittlichen Körnung (Partikelgrösse) von etwa 0.5 bis 5 mm, insbesondere etwa 1 bis 3 mm.

Vorteilhaft arbeitet man im Temperaturbereich oberhalb von 500° C, vorzugsweise bei etwa 550° bis etwa 600° C, und verdünnt die Iminodibenzyldämpfe mit einem inerten Trägergas, wie mit Stickstoff oder insbesondere der etwa 50- bis 200-fachen, beispielsweise der etwa 120- bis 140-fachen, molaren Menge Wasserdampf. Dabei arbeitet man vorteilhaft bei Normaldruck oder geringem Unterdruck, beispielsweise bei etwa 0.2 bis 1.1, insbesondere bei etwa 0.95 bis 1.05 bar (absolut) und lässt die Reaktionsgase auf Raumtemperatur abkühlen, wobei das gebildete Iminostilben und nicht umgesetztes Iminodibenzyl in fester, in dem kondensierten Transportwasser suspendierter Form anfällt. Der Iminostilbenanteil kann dann aus dem Kondensat abgetrennt und das nicht umgesetzte Iminodibenzyl isoliert und rezykliert werden. Die dabei anzuwendende Verfahrensweise ist bekannt und kann z.B. aus CH-442,319 entnommen werden.

Die nachfolgen Beispiele dienen zur Illustration der Erfindung; Temperaturen sind in Celsiusgraden, Druckwerte in bar absolut angegeben.

Beispiel 1: Die Dehydrierung von Iminodibenzyl (IDB) zu Iminostilben (IS) in der Gasphase wird in einem Rohrreaktor aus Quarzglas mit einem Durchmesser von 31 mm und einer Länge von 1 m durchgeführt. 30 g eines Katalysators mit folgenden Eigenschaften

| Zusammensetzung | 1.1 Gew.-% Chromoxid berechnet als $Cr_2O_3$ |
| | 22 Gew.-% einer Kaliumverbindung berechnet als $K_2O$ |
| | Rest Eisenoxid |
| Körnung | 1-2 mm |

wird zwischen zwei Füllkörperschichten (Korund; Körnung 1-2 mm) in der Mitte des Reaktionsrohres eingefüllt. Die obere Füllkörperschicht dient als Verdampfungszone für das Iminodibenzyl. 27.7 g/h Wasser werden in einem separaten Ofen verdampft und von oben in den Reaktor geleitet. 6 g/h Iminodibenzyl wird bei 120° C als Schmelze direkt in die Verdampferzone dosiert, mit dem vorerhitzten Dampf vermischt und verdampft. Durch die elektrische Beheizung des Reaktors wird das Dampfgemisch auf die Reaktionstemperatur von 550° C erhitzt. Nach der Umsetzung in der ca. 35 mm hohen Katalysatorschicht werden die Produktdämpfe in einem Gefäss auf Raumtemperatur abgekühlt, wodurch das Gemisch aus Iminodibenzyl und Iminostilben pulverförmig im kondensierten Wasser anfällt. Die zu verschiedenen Zeiten des Versuchs entnommenen Kondensate werden mit Dioxan homogenisiert und durch Kapillargaschromatographie analysiert (ohne Wasser). Während der Versuchsdauer von 860 Stunden ergab sich folgende Produktezusammensetzung (ohne Wasser)

| Zeit [h] | IDB Gew-% | IS Gew-% | Nebenverb.Gew-% | Umsatz % | Selektivität |
|----------|-----------|----------|-----------------|----------|--------------|
| 2 | 25.0 | 51.4 | 20.9 | 75 | 72 |
| 25 | 28.9 | 49.6 | 21.5 | 71 | 70 |
| 92 | 27.4 | 48.4 | 24.2 | 73 | 67 |
| 554 | 30.9 | 47.5 | 21.6 | 68 | 69 |
| 630 | 26.7 | 49.9 | 21.6 | 73 | 69 |
| 860 | 29.9 | 47.2 | 22.9 | 70 | 67 |

Neben Iminodibenzyl (IDB) und Iminostilben (IS) wird hauptsächlich 9-Methylacridin und Acridin als Nebenverbindungen festgestellt. Der Versuch wird während jeweils ca. 100 Stunden kontinuierlich durchgeführt und an den Wochenenden unterbrochen. Nach dem Unterbruch wird der Katalysator während einer Stunde mit Wasserdampf bei Reaktionstemperatur gespült und danach unter Stickstoff auf Raumtemperatur abgekühlt. Beim erneuten Anfahren wird in der umgekehrten Reihenfolge vorgegangen.

Beispiel 1 demonstriert die lange Lebensdauer und die kontinuierliche Fahrweise des Katalysators.

Beispiel 2: In einem Rohrreaktor aus Quarzglas gemäss Beispiel 1 werden 80 g eines Katalysators mit folgender Zusammensetzung eingefüllt:

EP 0 570 336 B1

| Zusammensetzung | 12.3 Gew.-% Kaliumoxid berechnet als $K_2O$ |
| | 1.9 Gew.-% Chromoxid berechnet als $Cr_2O_3$ |
| | 1.6 Gew.-% Wolframoxid berechnet als $WO_3$ |
| | 2.4 Gew.-% Ceroxid berechnet als $Ce_2O_3$ |
| | 1.5 Gew.-% Vanadiumoxid berechnet als $V_2O_5$ |
| | 0.3 Gew.-% Kobaltoxid berechnet als $Co_2O_3$ |
| Rest | Eisenoxid |
| Körnung | 1-2 mm |

Ein Strom von 40 g/h Iminodibenzyl und 480 g/h Wasserdampf wird bei 590° während 1145 h mit den in Beispiel 1 beschriebenen Wochenendunterbrüchen über den Katalysator geleitet. Das Produkt, welches durch Filtration vom Wasser getrennt wird, hat folgende Zusammensetzung (als Funktion der Reaktionszeit):

| Zeit [h] | IDB Gew-% | IS Gew-% | Nebenverb. Gew-% | Umsatz % | Selektivität % |
|---|---|---|---|---|---|
| 39 | 13.5 | 76.2 | 10.3 | 87 | 88 |
| 148 | 13.5 | 74.7 | 11.8 | 87 | 87 |
| 250 | 12.6 | 76.9 | 10.5 | 86 | 88 |
| 367 | 13.1 | 76.8 | 10.1 | 87 | 88 |
| 460 | 13.7 | 76.1 | 10.2 | 87 | 88 |
| 550 | 14.6 | 75.3 | 10.1 | 86 | 88 |
| 685 | 12.7 | 76.2 | 11.1 | 87 | 87 |
| 914 | 11.6 | 77.4 | 11.0 | 89 | 88 |
| 1028 | 13.0 | 75.8 | 11.2 | 87 | 87 |
| 1145 | 14.6 | 74.4 | 11.0 | 86 | 87 |

Beispiel 2 zeigt die gute Selektivität, die lange Lebensdauer von über 1100 h und die kontinuierliche Fahrweise eines Katalysators, enthaltend Wolfram-, Cer-, Vanadium- und Kobaltoxid als Promotoren.

Beispiele 3 bis 6: In der gleichen Versuchsanordnung und den gleichen Bedingungen wie im Beispiel 1 wird die Wassermenge im Verhältnis zur IDB-Dosierung über einen grossen Bereich variiert:

| Beispiel | Wasser [g/h] | IDB Gew-% | IS Gew-% | Nebenverb. Gew-% | Umsatz % | Selektivität % |
|---|---|---|---|---|---|---|
| 3 | 11.7 | 38.6 | 32.0 | 29.4 | 61 | 52 |
| 4 | 27.7 | 26.7 | 49.9 | 23.4 | 73 | 68 |
| 5 | 55.4 | 20.6 | 60.2 | 19.2 | 79 | 76 |
| 6 | 110.8 | 1.7 | 60.1 | 38.2 | 98 | 61 |

Die Beispiele 3 bis 6 zeigen den grossen Einfluss des Verhältnisses von Wasser zu IDB auf den Umsatz und die Selektivität der Reaktion.

Beispiele 7 bis 11: In der gleichen Versuchsanordnung und mit 30 g desselben Katalysators wie in Beispiel 1 wird die Dehydrierung bei 570° C durchgeführt. Dabei wird die Dosierung von IDB und Wasser so verändert, dass das Verhältnis Wasser zu IDB konstant bleibt.

| Beispiel | Wasser [g/h] | IDB [g/h] | IDB Gew-% | IS Gew-% | Nebenverb. Gew-% | Umsatz % | Selektivität % |
|---|---|---|---|---|---|---|---|
| 7 | 276.9 | 30 | 34.0 | 55.4 | 10.6 | 66 | 84 |
| 8 | 138.5 | 15 | 16.4 | 67.3 | 16.3 | 84 | 81 |
| 9 | 92.3 | 10 | 9.6 | 70.9 | 19.5 | 90 | 78 |
| 10 | 55.4 | 6 | 8.1 | 64.7 | 27.2 | 92 | 70 |
| 11 | 27.7 | 3 | 5.5 | 57.5 | 37.0 | 95 | 61 |

Beispiele 7 bis 11 demonstrieren den Einfluss der Kontaktzeit (Kontaktzeitfaktor = 1-10h

4

$$\text{(Kontaktzeitfaktor} = \frac{g_{(Katalysator)} \times h}{g_{(Iminodibenzyl)}})$$

pro Stunde)) bei gleichbleibendem Verhältnis von Wasser zu Iminodibenzyl (IDB) auf den Umsatz bzw. die Selektivität der Reaktion.

Beispiele 12 bis 15: In der gleichen Versuchsanordnung mit 30 g des gleichen Katalysators und denselben Dosierungen für Wasser und IDB wie in Beispiel 1 wird die Dehydrierungsreaktion bei unterschiedlichen Temperaturen durchgeführt. Die untenstehende Tabelle zeigt die Produktezusammensetzung

| Beispiel | Temperatur [°C] | IDB Gew-% | IS Gew-% | Nebenverb. Gew-% | Umsatz % | Selektivität % |
|----------|-----------------|-----------|----------|------------------|----------|----------------|
| 12 | 510 | 55.1 | 32.7 | 12.2 | 45 | 73 |
| 13 | 530 | 39.5 | 43.4 | 17.1 | 61 | 72 |
| 14 | 550 | 26.7 | 49.9 | 23.4 | 73 | 68 |
| 15 | 570 | 25.5 | 45.0 | 29.5 | 75 | 60 |

Die Beispiele 12 bis 15 zeigen den starken Einfluss der Temperatur auf die Umsetzungsgeschwindigkeit und die Selektivität der Reaktion.

Beispiel 16: In den Reaktor von Beispiel 1 werden 30 g eines Katalysators mit folgenden Eigenschaften eingefüllt:

| Zusammensetzung | 9.0 Gew.-% einer Kaliumverbindung berechnet als $K_2O$ |
|-----------------|--------------------------------------------------------|
| | 0.04 Gew.-% Chromoxid berechnet als $Cr_2O_3$ |
| | 2.7 Gew.-% einer Calziumverbindung berechnet als CaO |
| | 4.8 Gew.-% Ceroxid berechnet als $Ce_2O_3$ |
| | 2.5 Gew.-% Magnesiumoxid berechnet als MgO |
| | 2.0 Gew.-% Molybdänoxid berechnet als $MoO_3$ |
| | Rest Eisenoxid |
| Körnung | 1.5 mm |

Über den Katalysator wird bei 0.4 bar (absolut) und 570° C ein dampfförmiger Strom von 6.0 g/h IDB und 55.4 g/h Wasser geleitet. Das Produkt enthielt 2.9 Gew.-% IDB, 74.0 Gew.-% IS und 23.1 Gew.-% Nebenverbindungen (hauptsächlich Acridin und 9-Methylacridin) ohne Berücksichtigung des Wassers. Derselbe Versuch bei Normaldruck ergab eine Produktezusammensetzung von 6.2 Gew.-% IDB, 66.9 Gew.-% IS und 26.9 Gew.-% Nebenverbindungen.

Beispiel 16 zeigt den Einfluss von Unterdruck (Vakuum) auf den Umsatz und die Selektivität der Reaktion.

Beispiele 17 bis 20: In den folgenden Beispielen wird der Einfluss der Katalysatorzusammensetzung auf die Umsätze bzw. die Selektivitäten der Reaktion bei gleichen Versuchsbedingungen demonstriert. In der Versuchsapparatur von Beispiel 1 werden jeweils 30 g der untenstehenden Katalysatoren getestet (Körnung 1-2 mm). Bei 550° C werden 15 g/h IDB und 180 g/h Wasser dampfförmig über die Katalysatoren geleitet.

| Beispiel | IDB Gew-% | IS Gew-% | Nebenverb. Gew-% | Umsatz % | Selektivität % |
|----------|-----------|----------|------------------|----------|----------------|
| 17 | 28.2 | 59.0 | 12.8 | 72 | 82 |
| 18 | 18.0 | 67.7 | 14.3 | 82 | 83 |
| 19 | 20.9 | 63.2 | 15.9 | 79 | 80 |
| 20 | 27.2 | 60.0 | 12.8 | 73 | 82 |

Die Zusammensetzung der in den Beispielen 17 bis 20 verwendeten Katalysatoren ist in der nachfolgenden Tabelle gegeben:

| Beispiel | $Fe_2O_3$ [Gew.-%] | $K_2O$ [Gew.-%] | $Cr_2O_3$ [Gew.-%] | CaO [Gew.-%] | $Ce_2O_3$ [Gew.-%] | MgO [Gew.-%] | $MoO_3$ [Gew.-%] |
|----------|--------------------|-----------------|--------------------|--------------|--------------------|--------------|------------------|
| 17 | 61 | 22 | 1.1 | --- | --- | --- | --- |
| 18 | 85 | 9 | 1.9 | --- | --- | --- | --- |

(fortgesetzt)

| Beispiel | $Fe_2O_3$ [Gew.-%] | $K_2O$ [Gew.-%] | $Cr_2O_3$ [Gew.-%] | CaO [Gew.-%] | $Ce_2O_3$ [Gew.-%] | MgO [Gew.-%] | $MoO_3$ [Gew.-%] |
|---|---|---|---|---|---|---|---|
| 19 | 77 | 9 | 0.04 | 2.7 | 4.8 | 2.5 | 2.0 |
| 20 | 44 | 31 | 2.5 | --- | 0.5 | --- | 0.1 |

Die Beispiele 17 bis 20 zeigen, dass die Reaktion mit verschiedenen Katalysatoren mit stark unterschiedlicher Zusammensetzung durchgeführt werden kann.

Beispiel 21: In der Versuchsanordnung von Beispiel 1 werden 30 g eines Katalysators mit den folgenden Eigenschaften eingefüllt:

| Zusammensetzung | 1.3 Gew.-% Chromoxid berechnet als $Cr_2O_3$ |
|---|---|
| | 13.1 Gew.-% einer Kaliumverbindung berechnet als $K_2O$ |
| | Rest Eisenoxid |
| Körnung | 1-2 mm |

Bei 550° C und 1 bar absolut wird ein dampfförmiger Strom von 6 g/h Iminodibenzyl und 72 g/h Wasser über den Katalysator geleitet. Durch Filtration und Trocknung der auskondensierten Produkte kann eine Masse von 99 % bezogen auf die eingesetzte Substanzmenge (Iminodibenzyl) wiedergewonnen werden. Der Massenverlust von 1% ist auf die Abspaltung von Wasserstoff und Methan aus den Reaktionskomponenten zurückzuführen. Das isolierte Produkt hatte die Zusammensetzung: 76.2 Gew.-% Iminostilben, 3.8 Gew.-% Iminodibenzyl und 20 Gew.-% Nebenverbindungen. Die Gesamtausbeute an Iminostilben unter Berücksichtigung der Massenverluste beträgt somit 75.4 %.

Dieses Beispiel zeigt die hohen erreichbaren Ausbeuten und die geringen Massenverluste bei der kontinuierlichen Durchführung der Reaktion.

Vergleichsbeispiel 1: (Vergleich mit dem zyklischen Verfahren)

In der Versuchsanordnung von Beispiel 1 werden 50 g des in Beispiel 1 von CH-442,319 beschriebenen Katalysators eingefüllt. Gemäss den Angaben im oben genannten Patent wird die Reaktion bei 430° C, 1 bar absolut in zyklischer Fahrweise durchgeführt. Die Zykluszeiten betrugen: 5 Minuten Vorspülen mit Wasserdampf, 18 Minuten Reaktion, 5 Minuten Nachspülen mit Wasserdampf und 30 Minuten Regeneration (Oxidation des Katalysators mit Luft). Die Dosierungen betrugen: Vorspülen mit Wasserdampf: 38.3 g/h, Reaktion: 8.3 g/h IDB und 38.3 g/h Wasserdampf, Nachspülen: 38.3 g/h Wasserdampf und Regeneration: 100 ml/Minute Luft und 38.3 g/h Wasserdampf. Das während 20 Zyklen gesammelte Produkt wird abfiltriert und getrocknet. Die wiedergefundene Substanzmenge entsprach 88 Gew.-% der eingesetzten IDB-Menge. Da nach der Reaktionsphase ein Teil des Produktes auf dem Katalysator zurückbleibt, wird während der Regenerationsphase dieser adsorbierte Anteil mit Luft zu unbrauchbaren Produkten oxidiert. Die Produktzusammensetzung betrug im Durchschnitt: 65 Gew.-% Iminostilben, 28 Gew.-% Iminodibenzyl und 7 Gew.-% Nebenverbindungen. Die Gesamtausbeute unter Berücksichtigung der Massenverluste beträgt somit 57 %.

Dieses Vergleichsbeispiel zeigt die bedeutend schlechtere Ausbeute des zyklischen Verfahrens im Vergleich zum kontinuierlichen.

Vergleichsbeispiel 2: (Vergleich mit dem zyklischen Verfahren)

Mit der gleichen Versuchsanordnung, den gleichen Zykluszeiten, demselben Katalysator und denselben Bedingungen wie im Vergleichbeispiel 1 werden 5 g/h IDB und 23.3 g/h Wasserdampf über den Katalysator geleitet. Nach der Filtration und Trocknung der Reaktionsmasse kann noch 78 % der eingesetzten Substanzmenge isoliert werden. Die Produktzusammensetzung betrug im Durchschnitt: 72 Gew.-% Iminostilben, 16 Gew.-% Iminodibenzyl und 12 Gew.-% Nebenverbindungen. Die Gesamtausbeute unter Berücksichtigung der Massenverluste beträgt somit 56 %.

Dieses Vergleichsbeispiel zeigt, dass durch Verringerung des Verhältnisses von dosiertem IDB zur Katalysatormenge zwar der Umsatz erhöht werden kann, dass aber durch die grösseren Massenverluste bei der Regeneration des Katalysators die Gesamtausbeute nicht steigt.

**Patentansprüche**

1. Verfahren zur kontinuierlichen Herstellung von Iminostilben durch Hochtemperatur-Dehydrierung von Iminodibenzyl an einem Eisenoxid/Kaliumsalz-Kontaktkatalysator in der Dampfphase, dadurch gekennzeichnet, dass man einen Eisenoxid/Kaliumsalz-Kontaktkatalysator, enthaltend 35 bis 90 Gew.-% einer Eisenverbindung, berechnet als $Fe_2O_3$, und 7 bis 35 Gew.-% einer Kaliumverbindung, berechnet als $K_2O$, neben 0.0 bis 3.5 Gew.-% einer Chromverbindung, berechnet als $Cr_2O_3$ und gegebenenfalls üblichen Promotoren, verwendet, wobei der Promotorenanteil, bezogen auf den Kontaktkatalysator, höchstens 3.0 Gew.-% einer Calciumverbindung, berechnet als CaO, enthält.

2. Verfahren gemäss Anspruch 1, dadurch gekennzeichnet, dass man als Promotor eine Magnesium-, Cer-, Molybdän-, Kobalt-, Vanadium- oder Wolframverbindung verwendet.

3. Verfahren gemäss einem der Ansprüche 1 und 2, dadurch gekennzeichnet, dass man einen Eisenoxid/Kaliumsalz-Kontaktkatalysator, enthaltend 44 bis 85 Gew.-% einer Eisenverbindung, berechnet als $Fe_2O_3$, verwendet.

4. Verfahren gemäss einem der Ansprüche 1 bis 3, dadurch gekennzeichnet, dass man einen Eisenoxid/Kaliumsalz-Kontaktkatalysator, enthaltend 9 bis 31 Gew.-% einer Kaliumverbindung, berechnet als $K_2O$, verwendet.

5. Verfahren gemäss einem der Ansprüche 1 bis 4, dadurch gekennzeichnet, dass man den Kontaktkatalysator in einem adiabatischen Festbettreaktor oder einem Rohrbündel-, Fliessbett- und Wirbelschichtreactor in Kontakt mit Iminodibenzyldämpfen bringt.

6. Verfahren gemäss einem der Ansprüche 1 bis 5, dadurch gekennzeichnet, dass man den Kontaktkatalysator in Form von geometrischen Formkörpern von etwa 1 bis 6 mm Länge und etwa 0.5 bis 5 mm Durchmesser oder als Granulat mit einer durchschnittlichen Körnung (Partikelgrösse) von etwa 0.5 bis 5 mm einsetzt.

7. Verfahren gemäss einem der Ansprüche 1 bis 6, dadurch gekennzeichnet, dass man bei 500° bis 600°C arbeitet.

8. Verfahren gemäss einem der Ansprüch 1 bis 7, dadurch gekennzeichnet, dass man die Iminodibenzyldämpfe mit einem inerten Trägergas verdünnt.

9. Verfahren gemäss einem der Ansprüche 1 bis 7, dadurch gekennzeichnet, dass man die Iminodibenzyldämpfe mit der etwa 50- bis 200-fachen molaren Menge Wasserdampf verdünnt.

10. Verfahren gemäss einem der Ansprüche 1 bis 7, dadurch gekennzeichnet, dass man bei etwa 0.2 bis 1.1 bar (absolut) arbeitet.


**Claims**

1. A process for the continuous preparation of iminostilbene by high-temperature dehydrogenation of iminodibenzyl on an iron oxide/potassium salt contact catalyst in the vapour phase, wherein there is used an iron oxide/potassium salt contact catalyst comprising from 35 to 90 % by weight of an iron compound, calculated as $Fe_2O_3$, and from 7 to 35 % by weight of a potassium compound, calculated as $K_2O$, in addition to from 0.0 to 3.5 % by weight of a chromium compound, calculated as $Cr_2O_3$, and optionally customary promoters, the promoter component containing a maximum of 3.0 % by weight, based on the contact catalyst, of a calcium compound, calculated as CaO.

2. A process according to claim 1, wherein there is used as promoter a magnesium, cerium, molybdenum, cobalt, vanadium or tungsten compound.

3. A process according to either claim 1 or claim 2, wherein an iron oxide/potassium salt contact catalyst comprising from 44 to 85 % by weight of an iron compound, calculated as $Fe_2O_3$, is used.

4. A process according to any one of claims 1 to 3, wherein an iron oxide/potassium salt contact catalyst comprising from 9 to 31 % by weight of a potassium compound, calculated as $K_2O$, is used.

5. A process according to any one of claims 1 to 4, wherein the contact catalyst is brought into contact with iminodibenzyl vapours in an adiabatic fixed bed reactor or in a tube bundle reactor, fluid bed reactor or fluidised bed reactor.

6. A process according to any one of claims 1 to 5, wherein the contact catalyst is used in the form of geometric moulded bodies approximately 1 to 6 mm in length and approximately 0.5 to 5 mm in diameter or in the form of granules having an average particle size of about from 0.5 to 5 mm.

7. A process according to any one of claims 1 to 6, wherein the operation is carried out at from $500°$ to $600°$C.

8. A process according to any one of claims 1 to 7, wherein the iminodibenzyl vapours are diluted with an inert carrier gas.

9. A process according to any one of claims 1 to 7, wherein the iminodibenzyl vapours are diluted with an approximately 50- to 200-fold molar amount of water vapour.

10. A process according to any one of claims 1 to 7, wherein the operation is carried out at approximately from 0.2 to 1.1 bar (absolute).

**Revendications**

1. Un procédé de préparation en continu d'iminostilbène par déshydrogénation à température élevée d'iminodibenzyle sur un catalyseur de contact oxyde de fer/sel de potassium en phase vapeur, caractérisé en ce qu'on utilise un catalyseur de contact oxyde de fer/sel de potassium contenant de 35 à 90 % en poids d'un composé de fer, calculé comme $Fe_2O_3$ et de 7 à 35 % en poids d'un composé de potassium, calculé comme $K_2O$, en plus de 0,0 à 3,5% en poids d'un composé de chrome, calculé comme $Cr_2O_3$, et éventuellement des promoteurs habituels, la fraction des promoteurs par rapport au catalyseur de contact, contient au maximum 3,0% en poids d'un composé de calcium, calculé comme CaO.

2. Un procédé selon la revendication 1, caractérisé en ce qu'on utilise comme promoteur un composé de magnésium, de Cérium, de molybdène, de cobalt, de vanadium ou de tungstène.

3. Un procédé selon l'une quelconque des revendications 1 et 2, caractérisé en ce qu'on utilise un catalyseur de contact oxyde de fer/sel de potassium contenant de 44 à 85 % en poids d'un composé de fer, calculé comme $Fe_2O_3$.

4. Un procédé selon l'une quelconque des revendications 1 à 3, caractérisé en ce qu'on utilise un catalyseur de contact oxyde de fer/sel de potassium contenant de 9 à 31 % en poids d'un composé de potassium, calculé comme $K_2O$.

5. Un procédé selon l'une quelconque des revendications 1 à 4, caractérisé en ce que le catalyseur de contact est mis en contact avec les vapeurs d'iminodibenzyle dans un réacteur à lit fixe adiabatique ou dans un réacteur à faisceau de tubes, un réacteur à lit fluidisé et un réacteur à couche fluidisée.

6. Un procédé selon l'une quelconque des revendications 1 à 5, caractérisé en ce qu'on utilise le catalyseur de contact sous forme de corps moulés géométriques d'environ 1 à 6 mm de longueur et d'environ 0,5 à 5 mm de diamètre ou sous forme de granulés ayant une granulométrie moyenne (dimension des particules) d'environ 0,5 à 5 mm.

7. Un procédé selon l'une quelconque des revendications 1 à 6, caractérisé en ce qu'on opère entre $500°$ et $600°$C.

8. Un procédé selon l'une quelconque des revendications 1 à 7, caractérisé en ce qu'on dilue les vapeurs d'iminodibenzyle avec un gaz porteur inerte.

9. Un procédé selon l'une quelconque des revendications 1 à 7, caractérisé en ce qu'on dilue les vapeurs d'iminodibenzyle avec entre environ 50 et 200 fois la quantité molaire de vapeur d'eau.

10. Un procédé selon l'une quelconque des revendications 1 à 7, caractérisé en ce qu'on opère entre environ 0,2 et 1,1 bar (absolu).